# EUROPEAN PATENT SPECIFICATION

(11) **EP 0 341 885 B1**
(45) Date of publication and mention of the grant of the patent: **23.08.1995**
(21) Application number: 89304413.1
(22) Date of filing: 02.05.1989
(51) Int. Cl.: A01H 5/08, A01H 5/10, A01H 1/00, C12N 15/00

(54) **Tomatoes**
Tomaten
Tomates

(30) Priority: 11.05.1988 GB 8811115
(43) Date of publication of application: 15.11.1989
(73) Proprietor: ZENECA LIMITED, London W1Y 6LN (GB)
(72) Inventor: Bridges, Ian George, Slater Iowa 50244 (US); Schuch, Wolfgang Walter, Park Crowthorne (GB); Grierson, Donald, Shepshed Leicestershire (GB)
(74) Representative: Roberts, Timothy Wace

(56) References cited:
- EP-A- 0 240 208
- EP-A- 0 271 988
- WO-A-88/09334
- JOURNAL OF CELLULAR BIOCHEMISTRY, supplement 12C, 1988, ABSTRACTS OF THE 17THANNUAL MEETINGS, UCLA SYMPOSIA ON MOLECULAR &CELLULAR BIOLOGY, 28th February-10th April 1988, pages 126,147, abstract no. L
- 041, Alan R. Liss, Inc., New York, US; D. GRIERSON et al.: "Physiological andgenetic factors governing the expression of the polygalacturonase gene inripening tomatoes"
- JOURNAL OF CELLULAR BIOCHEMISTRY, supplement 12C, 1988, ABSTRACTS OF THE 17THANNUAL MEETINGS, UCLA SYMPOSIA ON MOLECULAR &CELLULAR BIOLOGY, 28th February-10th April 1988, pages 126,148, abstract no. L043, Alan R. Liss, Inc., New York, US; W.R. HIATT et al.: "Expression ofselected genes during tomato fruit maturation and ripening"

## Description

This invention relates to novel tomato fruit and seeds produced by genetic modification of the natural tomato plant.

Abstract L041 in Journal of Cellular Biochemistry (1988, Supplement 12C, page 147) discusses the ripening inhibitor (rin) tomato mutant, a naturally-occurring mutant in which less than 1% of the normal PG mRNA levels are produced. European patent application EP-A-240,208 (Calgene) describes polygalacturonase antisense constructs and suggests their use in plant transformation.

In our co-pending European patent specification No 87309853.7 (now published as 271988) we disclose tomato plants that have been genetically modified by inclusion of DNA constructs capable of expressing RNA complementary to RNA encoding fruit-softening enzymes. We have now shown that such tomato plants are able to produce fruit and fertile seed. Furthermore fruit of the selfed progeny of such plants show surprising properties, which may include an unexpectedly high level of inhibition of such fruit-softening enzymes.

According to the present invention we provide tomato fruit having a reduced level of expression of polygalacturonase resulting from the presence in the plant genome of DNA capable of generating antisense RNA complementary to the RNA that generates polygalacturonase. We further provide fertile seed, derived from such fruit, which may be cultured to produce similar fruit.

Fruit according to our invention may be obtained by growing and cropping, using conventional methods, the tomato plants described in our European Patent Application No 87309853.7.

Methods for making such plants, by genetic manipulation of known tomato plants, are fully described in the aforesaid European Patent Application No 271988 and in US Application Serial 119614, the complete disclosures of which applications are incorporated herein by reference.

A convenient method of obtaining such plants is to grow the seeds from tomato fruit according to the invention. We have deposited samples of such seeds with the National Collections of Industrial and Marine Bacteria (hereinafter NCIMB), Torry Research Station, PO Box 31, 135 Abbey Road, Ab- erdeen AB9 8DG, Scotland, under the reference numbers 40015 and 40134.

Fruit ripening is a complex physiological and biochemical process. It requires the activation of specific genes which are required to give fruit the desirable characteristics important for human consumption, and for industrial processing for consumption. In tomatoes, the end product of the mature fruit is characterised by good, intense and even colour production, specific flavour components and texture suitable for the intended end use ( consumption fresh or industrial processing). In biochemical terms these attributes are brought about through the action of enzymes that are responsible for the breakdown of chlorophyll and the synthesis of lycopene ,8-carotene for colour production, the synthesis of flavour components through lypoxygenase, the synthesis of enzymes involved in the maintenance of water and mineral balance, enzymes controlling vitamin production, and most importantly for the purposes of the present invention, the production of cell wall degrading enzymes leading to fruit softening.

Fruit according to our invention show reduced levels of expression of the polygalacturonase gene. This results from the presence in the genome of one, or several, antisense constructs to the gene.

Both pectinesterase and polygalacturonase are involved in the biochemical conversion of pectic cell wall substances during fruit ripening. Pectins demethylated by pectinesterase are believed to be the substrate for polygalacturonase. According to one hypothesis, polygalacturonase solubilises pectic fragments during early ripening stages, and reduces the molecular weight of pectic fragments during later stages of ripening. In fruit according to the invention, the reduction in molecular weight of solubilised pectin is inhibited.

Preliminary results indicate that tomatoes of the invention having a reduced level of expression of polygalacturonase retain their firmness for a longer period after harvesting than similar tomatoes having normal levels of expression of fruit softening enzymes. They are consequently expected to soften more slowly on the plant, be harder at the time of harvesting and have a longer shelf life, with potentially increased resistance to infection. It is useful to be able to harvest fruit later when flavour, aroma and colour may have developed more fully. Fruit according to our invention may also show increased solids content and altered pectin and cell wall components, with consequent processing advantages. These advantages include energy savings during tomato puree production, increased insoluble solids, and improved gelling qualities and colour of puree.

Seeds according to our invention may be obtained from fruit according to our invention by conventional methods. For example, tomato seeds are separated from the pulp of the ripe fruit and dried, following which they may be stored for one or more seasons.

The fruit of our invention may be sold for immediate consumption, raw or cooked, or processed by canning or conversion to soup, sauce or paste. Equally, they may be used to provide seeds according to the invention.

The tomato plants described in European Patent Application No 87309853.7 are heterozygous for the antisense constructs. The seeds obtained from self fertilisation of such plants are a population in which the antisense constructs behave like single Mendelian genes and are distributed according to Mendelian principles: eg, where such a plant contains only one copy of the construct, 25% of the seeds contain two copies of the construct, 50 % contain one copy and 25% contain no copy at all. Thus not all the offspring of selfed tomato plants according to European Patent Application No 87309853.7 produce fruit and seeds according to the present invention, and those which do may themselves be either heterozygous or homozygous for the defining trait. Some homozygous fruit according to the invention (eg those obtained from NCIB 40134) are found to show extremely low levels of expression of the fruit-ripening enzyme, eg 5% or less as compared with similar known fruit.

It is convenient to maintain a stock of seed which is homozygous for the antisense construct. All crosses of such seed stock will contain at least one copy of the construct, and self-fertilized progeny will contain two copies, i.e. be homozygous in respect of the character. Such homozygous seed stock may be conventionally used as one parent in F1 crosses to produce heterozygous seed for marketing. Such seed, and fruit derived from it, form further aspects of our invention. It is possible according to our invention to transform two or more plants with different antisense constructs and to cross the progeny of the resulting lines, so as to obtain seed of plants which contain two or more antisense constructs leading to reduced expression of polygalacturonase.

The following examples illustrate our invention.

### EXAMPLE 1

Example 40 of our European Patent Application No 87309853.7 describes the preparation of a tomato plant containing the PG antisense vector pJR16A incorporated in its genome. This plant (referenced as GR16, and derived from Lycoper- sicon esculentum Mill cv Ailsa Craig) was grown in a growth room under standard conditions suitable for growing tomatoes. The flowers were self-fer- tilised, and 15 resulting fruit collected. Analysis of these by the method of G A Tucker, N G Robertson, D Grierson, (European Journal of Biochemistry, 112 p 119-124, 1980) indicated a low level of expression of the polygalacturonase enzyme, approximately 10% of that in corresponding conventional tomato fruit at similar stage of development. Fruit were also tested for firmness, and showed increased firmness. throughout ripening.Seeds from the fruit were separated from the pulp, allowed to dry in air at room temperature (about 20 °C) and stored dry in brown paper envelopes at room temperature. About 400 of the resulting seeds were deposited with the NCIMB under reference 40015.

### EXAMPLE 2

Twelve seeds produced as in Example 1 were taken, grown to maturity and self-fertilized. Plant tissue from each of the 12 resulting seedlings was tested by Southern blot analysis as described in experiment 40 of European Patent Application No 87309853.7. Homozygotic and heterozygotic plants were identified. Seed from homozygous plants was collected, appropriately labelled and stored for future use. Seeds of one such plant, coded GR 105, was deposited with the NCIMB under reference 40134. Analysis of fruit of the plant GR 105 by the method of G A Tucker, N G Robertson, D Grierson, (European Journal of Biochemistry, 112 p 119-124, 1980) indicated an exceptionally low level of expression of the polygalacturonase enzyme, less than 1 % of that in corresponding conventional tomato fruit at a similar stage of development.

Another way of obtaining homozygous seed would be to backcross GR16, or a similar transformant, or offspring thereof, to the untransformed Ailsa Craig variety. Standard genetic analysis then permits identification of parent seed homozygous for the antisense construct.

## Claims

1. A genetically modified tomato fruit which is homozygous for a polygalacturonase antisense construct and has a reduced level of expression of polygalacturonase resulting from the presence in the plant genome of DNA capable of generating antisense RNA complementary to polygalacturonase mRNA, where the reduced level of expression of polygalacturonase is less than 1% of that of similar unmodified tomatoes at a corresponding development stage.

2. A tomato seed which is homozygous for a polygalacturonase antisense construct and from which a fruit as claimed in claim 1 is derivable.

3. Use of a tomato plant capable of bearing fruit as claimed in claim 1 as one parent in an F1 cross to produce F1 hybrids.

4. A process of producing F1 hybrid tomato seed which comprises producing a plant capable of bearing genetically modified tomato fruit as claimed in claim 1, crossing such a plant with a second homozygous tomato variey, and recovering F1 hybrid seed.

## Patentansprüche

1. Genetisch modifizierte Tomatenfrucht, die für ein Polygalacturonase-Antisinn-Konstrukt homozygot ist und eine Verringerte Polygalacturonase-Expression aufweist, die sich durch die Gegenwart von DNA in dem Pflanzen-Genom ergibt, welche Antisinn-RNA erzeugen kann, die komplementär zur PolygalacturonasemRNA ist, wobei die verringerte Polygalacturonase-Expression weniger als 1 % von der ähnlicher unmodifizierter Tomaten in einem entsprechenden Entwicklungsstadium beträgt.

2. Tomatensamen, der für ein Polygalacturonase-Antisinn-Konstrukt homozygot ist, und von dem eine Frucht nach Anspruch 1 erhältlich ist.

3. Verwendung einer Tomatenpflanze, die Früchte nach Anspruch 1 tragen kann, als ein Elternteil in einer F1-Kreuzung zur Produktion von F1-Hybriden.

4. Verfahren zur Produktion von FI-Hybrid-Tomatensamen, bei dem eine Pflanze, die eine genetisch modifizierte Tomatenfrucht nach Anspruch 1 tragen kann, produziert wird, eine derartige Pflanze mit einer zweiten homozygoten Tomatenvarietät gekreuzt und der F1-Hybrid-Samen gewonnen wird.

## Revendications

1. Fruit de tomate génétiquement modifié qui est homozygote pour un produit d'assemblage antisens de polygalacturonase et qui possède un taux réduit d'expression de polygalacturonase résultant de la présence dans le génome de plante d'un ADN capable d'engendrer un ARN antisens complémentaire de l'ARNm de polygalacturonase, le taux réduit d'expression de polygalacturonase étant inférieur à 1 % de celui de tomates non modifiées similaires à un stade de développement correspondant.

2. Semence de tomate qui est homozygote pour un produit d'assemblage antisens de polygalacturonase, et à partir de laquelle peut être obtenu un fruit suivant la revendication 1.

3. Utilisation d'un plan de tomate pouvant porter un fruit suivant la revendication 1 comme parent dans un croisement F1 pour produire des hybrides F1.

4. Procédé de production d'une semence de tomate hybride F1, qui comprend la production d'un plant pouvant porter un fruit de tomate génétiquement modifié suivant la revendication 1, le croisement d'un tel plant avec une seconde variété de tomate homozygote, et la récolte des semences hybrides F1.
